# EUROPEAN PATENT SPECIFICATION

(11) **EP 3 471 686 B1**
(45) Date of publication and mention of the grant of the patent: **18.06.2025**
(21) Application number: 17732194.0
(22) Date of filing: 19.06.2017
(51) Int. Cl.: A61K 6/35

(54) **DENTURE ADHESIVE**
ZAHNPROTHESENHAFTMITTEL
ADHÉSIF POUR PROTHÈSE DENTAIRE

(30) Priority: 20.06.2016 GB 201610707
(43) Date of publication of application: 24.04.2019
(73) Proprietor: Reckitt Benckiser Health Limited, Slough, Berkshire SL1 3UH (GB)
(72) Inventor: CRABB, James, Leeds Yorkshire LS15 4JE (GB); HEATON, Frances, Hull Humberside HU8 7DS (GB); ROPIC-LEACH, Melanie, Hull Humberside HU8 7DS (GB)
(74) Representative: Hodgetts, Catherine Dawn
(86) International application number: PCT/GB2017/051774
(87) International publication number: WO 2017/220977

(56) References cited:
- WO-A1-80/01166
- US-A1- 2009 239 972
- US-A1- 2010 119 468

## Description

The present invention relates to a denture adhesive composition.

Denture adhesive compositions are required to adhere a denture to the gum. Proper adhesion is required so that the denture can be sued effective without providing discomfort for the user by allowing slipping or by allowing food residue to penetrate beneath the denture.

US2009/239972A1 describes the use of petrolatum and paraffinium liquidum / mineral oil in denture adhesive compositions. This document does not disclose the use of a C5 to C15 polyalkene.

There is a continued need for improved denture adhesive compositions.

According to a first aspect of the invention there is provided a denture adhesive according to claim 1.

Preferably the C5 to C15 polyalkene comprises a polyalphaolefin. For clarity the term C5 to C15 polyalkene refers to the chain length of the monomers which make up the polymeric polyalkene.

With the use of the at least one component comprising a C5 to C15 polyalkene it has been found that effective / advantageous adhesive compositions may be provided.

A principle advantage is that the composition provides a high level of "standing" viscosity and in particular that they can be easily discharged from the tubes in which the denture adhesive compositions are typically contained / sold. Thus preferably the compositions are shear-thinning. Most preferably, the denture adhesive composition is free of mineral oils and polymers comprising C2-C4 species, especially C2 (polyethylene or polymers comprising ethylene).

A secondary advantage associated with the compositions of the present invention is that the detrimental properties of these entities can be ameliorated or even avoided.

### C5 to C15 polyalkene

The at least one component comprising the C5 to C15 polyalkene is present in the composition in a total amount of from 30 to 50% based on the total weight of the composition. Most preferably the total amount of C5 to C15 polyalkene present in the composition is in the range of from 35-45%wt.

The at least one component comprising the C5 to C15 polyalkene may be a gel or an oil component.

Most preferably the compositions comprise both a gel component comprising C5 to C15 polyalkene and an oil component comprising C5 to C15 polyalkene.

It is preferred that the composition comprises C5 to C15 polyalkene in a gel component form.

It is also preferred that the composition comprises C5 to C15 polyalkene in an oil from. especially 15 to 20%wt based on the total weight of the composition.

It is especially preferred that the compositions of the invention comprise C5 to C15 polyalkene in a gel component form and also C5 to C15 polyalkene in an oil form.

It is preferred that the gel form comprises the C5 to C15 polyalkene together with the viscosity index improver as a pre-mixed ingredient. In this case it is preferred that the viscosity index improver comprises a hydrogenated styrene isoprene copolymer.

It is preferred that the oil form comprises only the C5 to C15 polyalkene.

Preferably the C5 to C15 polyalkene is in at least partially and most preferably fully hydrogenated form. Where both a gel and an oil form of the C5 to C15 polyalkene are present in the composition it is preferred that both are in at least partially and most preferably fully hydrogenated form.

Generally the C5 to C15 polyalkene has a molecular weight between 300 and 1500g/mol, and especially 400 and 1000 g/mol. Suitable alkenes for producing the polyalkene include pentene, hexene, heptene, octene, nonene, decene, undodecene and dodecene, preferably decene.

A particularly preferable polyalphaolefin is food grade polydecene, especially hydrogenated polydecene and most preferably fully hydrogenated polydecene.

### Viscosity Index Improver

By the term "viscosity index improver" is meant a material which is capable of thickening a C5 to C15 polyalkene oil but which does not act as an adhesion promotor in the composition.

The viscosity index improver is present in an amount from 0.001% to 20.0% by weight of the composition. Most preferably the viscosity index improver is present in an amount of 0.5% to 10%wt, such as 1% to 7 %wt and most preferably 2 to 5 %wt of the composition.

The viscosity index improver is selected from the group consisting of polymethacrylates, olefin copolymers, hydrogenated styrene-diene copolymers, styrene polyesters, rubber, polyvinylchloride, nylon, fluorocarbon, polyurethane prepolymer, polyethylene, polystyrene, polypropylene, acrylic resins, microcrystalline wax, elastomers, poly(alkyl fumarate co-vinyl acetate), alkylated polystyrene, poly(t-butyl styrene), and combinations thereof.

Most preferably the viscosity index improver comprises a hydrogenated styrene isoprene copolymer.

Preferably in the gel component comprising C5 to C15 polyalkene and the viscosity index improver, the ratio of the C5 to C15 polyalkene and the viscosity index improver is in the range of from 20:1 to 5:1 by weight.

### Denture adhesive component

The compositions of the invention comprise a denture adhesive component. The adhesion promotor aids the adhesion of the compositions to the gums and/or to the dentures.

The denture adhesive composition comprises a denture adhesive component selected from the group consisting of cellulose, poly(n-butyl vinyl ether), poly(styrene-co-maleic anhydride), starch, saccharide, polyethylene oxides, polyethylene glycols, polyvinyl alcohols, carrageenan, alginates, karaya gum, xanthan gum, guar gum, gelatin, algin, tragacanth, chitosan, acrylamide polymers, carboxypolymethylene, polyamines, polyquaternary compounds, polyvinylpyrrolidone, polylvinylalkylacrylates /maleic anhydride copolymers and salts thereof including mixed salts, polymeric acids, polymeric salts, polyhydroxy compounds, and combinations thereof.

An especially preferred adhesion promotor is a polyvinylmethacrylate/maleic anhydride copolymer, especially the mixed calcium/sodium salt thereof. Carboxymethylcellulose, salts of carboxymethylcellulose are also preferred adhesion promotors. A combination to polyvinylmethacrylate/maleic anhydride copolymer and a carboxymethylcellulose, especially sodium carboxymethylcellulose is especially preferred as an adhesion promotor.

The denture adhesive component is present in an amount from to 65%wt based on the total weight of the composition.

The composition may contain other adjunct materials such as fillers, pH adjusting agents, preservatives, dyes / colourants, flavourings.

Preferably the denture adhesive composition can be dispensed from a tube by hand.

An especially preferred denture adhesive composition according to the present invention comprises;
a) a C5 to C15 polyalkene oil, and
b) a gel component comprising a C5 to C15 polyalkene oil and a viscosity index improver.

Preferably this composition comprises hydrogenated C5 to C15 polyalkene in both the oil and the gel, and most preferably fully hydrogenated C5 to C15 polyalkene. It is especially preferred that the gel component comprises hydrogenated styrene isoprene copolymer as the viscosity index improver. It is also preferred that this denture adhesive composition can be dispensed from a tube.

Unless stated otherwise, all amounts herein are stated as percentages by weight based on the total weight of the composition.

According to a second aspect of the invention there is provided a method of preparing a denture adhesive composition according to claim 10.

Preferred compositions according the invention are given in the tables below. Further examples within the scope of the invention will be apparent to the person skilled in the art. All amounts are given as percentages by weight based on the total weight of the composition.

**Table 1;**

| **Ingredient** | **Ex 1** | **Ex 2** | **Ex 3** |
|---|---|---|---|
| Calcium/Sodium polyvinylmethacrylate/maleic anhydride (PVM/MA) copolymer | 31% | 31% | 31% |
| Sodium Carboxymethylcellulose | 23% | 23% | 23% |
| Hydrogenated Polydecene (>85%) Hydrogenated Styrene Isoprene Copolymer (about 15%) Tocopherol (<0.1%) | 28% | 28% | 28% |
| Hydrogenated Polydecene | 17.8740% | 17.88% | 18% |
| Iron(III) oxide | 0.006% | 0% | 0% |
| L-Menthol, Menthone, 1,8-Cineol, DIPENTENE, Menthyl acetate | 0.1% | 0.1% | 0% |
| L-Menthol | 0.02% | 0.02% | 0% |
| Soy oil (82.35%), Chamomile extract (17.5%), BHT (0.15%), alpha-bisabolol | 0% | 0% | 0% |
| | 100.000% | 100.000% | 100.000% |

**Table 2;**

| **Ingredient** | **Ex 4** | **Ex 5** | **Ex 6** |
|---|---|---|---|
| Calcium/Sodium polyvinylmethacrylate/maleic anhydride (PVM/MA) copolymer | 21% | 21% | 21% |
| Sodium Carboxymethylcellulose | 33% | 33% | 33% |
| Hydrogenated Polydecene (>85%) Hydrogenated Styrene Isoprene Copolymer (about 15%) Tocopherol (<0.1%) | 26% | 26% | 26% |
| Hydrogenated Polydecene | 17.874% | 19.874% | 19.88% |
| Iron(III) oxide | 0.006% | 0.006% | 0% |
| L-Menthol, Menthone, 1,8-Cineol, DIPENTENE, Menthyl acetate | 0.1% | 0.1% | 0.1% |
| L-Menthol | 0.02% | 0.02% | 0.02% |
| Soy oil (82.35%), Chamomile extract (17.5%), BHT (0.15%), alpha-bisabolol | 2% | 0% | 0% |
| | 100.000% | 100.000% | 100.000% |

## Claims

1. A denture adhesive composition, comprising:
a) at least one component comprising a C5 to C15 polyalkene present in the composition in an amount of from 30% to 50%wt based on the total weight of the composition; and
b) a viscosity index improver selected from the group consisting of polymethacrylates, olefin copolymers, hydrogenated styrene-diene copolymers, styrene polyesters, rubber, polyvinylchloride, nylon, fluorocarbon, polyurethane prepolymer, polyethylene, polystyrene, polypropylene, acrylic resins, microcrystalline wax, elastomers, poly(alkyl fumarate co-vinyl acetate), alkylated polystyrene, poly(t-butyl styrene), and combinations thereof, wherein the viscosity index improver is present in an amount from 0.001% to 20.0%wt;
wherein the composition comprises a denture adhesive component;
wherein the denture adhesive component is selected from the group consisting of cellulose, poly(n-butyl vinyl ether), poly(styrene-co-maleic anhydride), starch, saccharide, polyethylene oxides, polyethylene glycols, polyvinyl alcohols, carrageenan, alginates, karaya gum, xanthan gum, guar gum, gelatin, algin, tragacanth, chitosan, acrylamide polymers, carboxypolymethylene, polyamines, polyquaternary compounds, polyvinylpyrrolidone, polylvinylalkylacrylates /maleic anhydride copolymers and salts thereof including mixed salts, polymeric acids, polymeric salts, polyhydroxy compounds, and combinations thereof; and
wherein the denture adhesive component is present in an amount from 40% to 65% based on the total weight of the composition.

2. A denture adhesive composition according to claim 1, wherein the C5 to C15 polyalkene comprises a polydecene.

3. A denture adhesive composition according to claim 1 or 2, wherein the composition comprises the C5 to C15 polyalkene as a gel component.

4. A denture adhesive composition according to any one of the preceding claims wherein the composition comprises the C5 to C15 polyalkene as an oil.

5. A denture adhesive composition according to either one of claims 3 or 4 wherein the composition comprises the C5 to C15 polyalkene as both a gel component and an oil.

6. A denture adhesive composition according to any one of the preceding claims, wherein the viscosity index improver is in an amount of from 0.5% to 10%wt.

7. A denture adhesive composition according to any one of the preceding claims, wherein the hydrogenated styrene diene copolymer comprises hydrogenated styrene-isoprene copolymer.

8. A denture adhesive composition according to any preceding claim, wherein the denture adhesive component comprises polylvinylalkylacrylates /maleic anhydride copolymers, salts including mixed salts thereof, carboxymethylcellulose, salts of carboxymethylcellulose or a combination thereof.

9. A denture adhesive composition according to any of the preceding claims, wherein the denture adhesive composition can be dispensed from a tube by hand.

10. A method of preparing a denture adhesive composition according to any one of the preceding claims, wherein an oil component comprising a C5 to C15 polyalkene is pre-mixed with the viscosity index improver before being added to the remaining ingredients of the composition.

11. A method according to claim 10, wherein the ratio of the oil component comprising a C5 to C15 polyalkene and the viscosity index improver is from 20:1 to 5:1 by weight.

## Patentansprüche

1. Zahnprothesenhaftmittel-Zusammensetzung, umfassend:
a) mindestens eine Komponente, die ein C5- bis C15-Polyalken umfasst, das in der Zusammensetzung in einer Menge von 30 bis 50 Gew.-%, basierend auf dem Gesamtgewicht der Zusammensetzung, vorhanden ist; und
b) einen Viskositätsindexverbesserer, ausgewählt aus der Gruppe bestehend aus Polymethacrylaten, Olefincopolymeren, hydrierten Styrol-Dien-Copolymeren, Styrolpolyestern, Kautschuk, Polyvinylchlorid, Nylon, Fluorkohlenstoff, Polyurethan-Prepolymer, Polyethylen, Polystyrol, Polypropylen, Acrylharzen, mikrokristallinem Wachs, Elastomeren, Poly(alkylfumarat-co-vinylacetat), alkyliertem Polystyrol, Poly(t-butylstyrol) und Kombinationen davon, wobei der Viskositätsindexverbesserer in einer Menge von 0,001 Gew.-% bis 20,0 Gew.-% vorhanden ist;
wobei die Zusammensetzung eine Zahnprothesenhaftmittel-Komponente umfasst;
wobei die Zahnprothesenhaftmittel-Komponente ausgewählt ist aus der Gruppe bestehend aus Cellulose, Poly(n-butylvinylether), Poly(styrol-co-maleinsäureanhydrid), Stärke, Saccharid, Polyethylenoxiden, Polyethylenglykolen, Polyvinylalkoholen, Carrageen, Alginaten, Karayagummi, Xanthangummi, Guargummi, Gelatine, Algin, Tragant, Chitosan, Acrylamid-Polymeren, Carboxypolymethylen, Polyaminen, polyquaternären Verbindungen, Polyvinylpyrrolidon, Polylvinylalkylacrylat/Maleinsäureanhydrid-Copolymeren und deren Salzen, einschließlich Mischsalzen, Polymersäuren, Polymersalzen, Polyhydroxyverbindungen, und Kombinationen davon; und
wobei die Zahnprothesenhaftmittel-Komponente in einer Menge von 40 % bis 65 %, basierend auf dem Gesamtgewicht der Zusammensetzung, vorhanden ist.

2. Zahnprothesenhaftmittel-Zusammensetzung nach Anspruch 1, wobei das C5- bis C15-Polyalken ein Polydecen umfasst.

3. Zahnprothesenhaftmittel-Zusammensetzung nach Anspruch 1 oder 2, wobei die Zusammensetzung das C5- bis C15-Polyalken als eine Gelkomponente umfasst.

4. Zahnprothesenhaftmittel-Zusammensetzung nach einem der vorhergehenden Ansprüche, wobei die Zusammensetzung das C5- bis C15-Polyalken als ein Öl umfasst.

5. Zahnprothesenhaftmittel-Zusammensetzung nach Anspruch 3 oder 4, wobei die Zusammensetzung das C5- bis C15-Polyalken sowohl als eine Gelkomponente als auch als ein Öl umfasst.

6. Zahnprothesenhaftmittel-Zusammensetzung nach einem der vorhergehenden Ansprüche, wobei der Viskositätsindexverbesserer in einer Menge von 0,5 bis 10 Gew.-% vorhanden ist.

7. Zahnprothesenhaftmittel-Zusammensetzung nach einem der vorhergehenden Ansprüche, wobei das hydrierte Styrol-Dien-Copolymer hydriertes Styrol-Isopren-Copolymer umfasst.

8. Zahnprothesenhaftmittel-Zusammensetzung nach einem der vorhergehenden Ansprüche, wobei die Zahnprothesenhaftmittel-Komponente Polylvinylalkylacrylat/Maleinsäureanhydrid-Copolymere, Salze einschließlich deren Mischsalzen, Carboxymethylcellulose, Salze von Carboxymethylcellulose oder eine Kombination davon umfasst.

9. Zahnprothesenhaftmittel-Zusammensetzung nach einem der vorhergehenden Ansprüche, wobei die Zahnprothesenhaftmittel-Zusammensetzung von Hand aus einer Tube abgegeben werden kann.

10. Verfahren für das Zubereiten einer Zahnprothesenhaftmittel-Zusammensetzung nach einem der vorhergehenden Ansprüche, wobei eine Ölkomponente, die ein C5-bis C15-Polyalken umfasst, mit dem Viskositätsindexverbesserer vorgemischt wird, bevor sie zu den restlichen Inhaltsstoffen der Zusammensetzung zugegeben wird.

11. Verfahren nach Anspruch 10, wobei das Verhältnis der Ölkomponente, die ein C5-bis C15-Polyalken umfasst, und des Viskositätsindexverbesserers von 20:1 bis 5:1 nach Gewicht beträgt.

## Revendications

1. Composition adhésive pour prothèse dentaire, comprenant :
a) au moins un constituant comprenant un polyalcène C5 à C15, présent dans la composition à une teneur comprise entre 30 % et 50 % en poids par rapport au poids total de la composition ; et
b) un modificateur d'indice de viscosité sélectionné parmi le groupe constitué de polyméthacrylates, copolymères d'oléfines, copolymères hydrogénés styrène-diène, polyesters de styrène, caoutchouc, polychlorure de vinyle, nylon, fluorocarbone, prépolymère de polyuréthane, polyéthylène, polystyrène, polypropylène, résines acrylique, cire microcristalline, élastomères, poly(alkyl fumarate-co-acétate de vinyle), polystyrène alkylé, poly(t-butylstyrène) et leurs combinaisons, dans lequel le modificateur d'indice de viscosité est présent à une teneur allant de 0,001 % à 20,0 % en poids ;
dans laquelle la composition comprend un constituant adhésif pour prothèse dentaire ;
dans laquelle le constituant adhésif pour prothèse dentaire est sélectionné parmi le groupe constitué de cellulose, poly(éther vinylique de n-butyle), poly(styrène-co-anhydride maléique), amidon, saccharide, oxydes de polyéthylène, polyéthylène glycol, alcools polyvinyliques, carraghénane, alginates, gomme karaya, gomme xanthane, gomme guar, gélatine, algine, tragacanthe, chitosane, polymères d'acrylamide, carboxypolyméthylène, polyamines, composés polyquaternaires, polyvinylpyrrolidone, copolymères de polyvinylalkylacrylates / anhydride maléique et leurs sels y compris les sels mixtes, acides polymériques, sels polymériques, composés polyhydroxylés, et leurs combinaisons ; et
dans laquelle le constituant adhésif pour prothèse dentaire est présent à une teneur allant de 40 % à 65 % par rapport au poids total de la composition.

2. Composition adhésive pour prothèse dentaire selon la revendication 1, dans laquelle le polyalcène C5 à C15 comprend un polydécène.

3. Composition adhésive pour prothèse dentaire selon la revendication 1 ou 2, dans laquelle la composition comprend le polyalcène C5 à C15 en tant que constituant de gel.

4. Composition adhésive pour prothèse dentaire selon l'une quelconque des revendications précédentes, dans laquelle la composition comprend le polyalcène C5 à C15 en tant qu'huile.

5. Composition adhésive pour prothèse dentaire selon l'une ou l'autre des revendications 3 ou 4, dans laquelle la composition comprend le polyalcène C5 à C15 à la fois en tant que constituant de gel et en tant qu'huile.

6. Composition adhésive pour prothèse dentaire selon l'une quelconque des revendications précédentes, dans laquelle le modificateur d'indice de viscosité est présent à une teneur comprise entre 0,5 % et 10 % en poids.

7. Composition adhésive pour prothèse dentaire selon l'une quelconque des revendications précédentes, dans laquelle le copolymère hydrogéné styrène-diène comprend un copolymère styrène-isoprène hydrogéné.

8. Composition adhésive pour prothèse dentaire selon l'une quelconque des revendications précédentes, dans laquelle le constituant adhésif pour prothèse dentaire comprend des copolymères de polyvinylalkylacrylates / anhydride maléique, leurs sels y compris les sels mixtes, carboxyméthylcellulose, sels de carboxyméthylcellulose, ou une combinaison de ceux-ci.

9. Composition adhésive pour prothèse dentaire selon l'une quelconque des revendications précédentes, dans laquelle la composition adhésive pour prothèse dentaire peut être délivrée manuellement depuis un tube.

10. Procédé de préparation d'une composition adhésive pour prothèse dentaire selon l'une quelconque des revendications précédentes, dans lequel un constituant huileux comprenant un polyalcène C5 à C15 est prémélangé avec le modificateur d'indice de viscosité avant d'être ajouté aux autres ingrédients de la composition.

11. Procédé selon la revendication 10, dans lequel le rapport entre le constituant huileux comprenant un polyalcène en C5 à C15 et le modificateur d'indice de viscosité est compris entre 20:1 et 5:1 en poids.
